# EUROPÄISCHE PATENTSCHRIFT

(11) **EP 2 812 036 B1**
(45) Veröffentlichungstag und Bekanntmachung des Hinweises auf die Patenterteilung: **22.07.2015**
(21) Anmeldenummer: 14722650.0
(22) Anmeldetag: 06.05.2014
(51) Int. Cl.: G01N 31/22, A61L 2/28

(54) **STERILANZEIGE DURCH PHASENWECHSEL**
STERILE STATUS INDICATOR BY MEANS OF PHASE CHANGE
INDICATEUR DE STÉRILITÉ PAR CHANGEMENT DE PHASE

(30) Priorität: 07.05.2013 DE 102013208332
(43) Veröffentlichungstag der Anmeldung: 17.12.2014
(73) Patentinhaber: Aesculap AG, 78532 Tuttlingen (DE)
(72) Erfinder: MOTZ, Corvin, 88630 Pfullendorf (DE); ZIERIS, Gerold, 78570 Mülheim (DE); AMANN, Joachim, 78357 Mühlingen-Zoznegg (DE)
(74) Vertreter: Winter, Brandl, Fürniss, Hübner, Röss, Kaiser, Polte - Partnerschaft mbB
(86) Internationale Anmeldenummer: PCT/EP2014/059242
(87) Internationale Veröffentlichungsnummer: WO 2014/180849

(56) Entgegenhaltungen:
- WO-A1-01/86289
- WO-A2-2008/041146
- WO-A2-2012/135694
- US-A1- 2012 095 605

## Beschreibung

Die vorliegende Erfindung betrifft die Verwendung eines Phasenwechselmaterials zur Herstellung einer Sterilanzeige für einen Sterilisationscontainer sowie einen Sterilisationscontainer mit einer Sterilanzeige, die auf diesem Prinzip beruht.

Im Stand der Technik sind zahlreiche Formen von Sterilanzeigen bzw. Sterilindikatoren für Sterilisationscontainer bekannt. Solche Indikatoren dienen dazu, dem Personal anzuzeigen, ob ein Sterilisationscontainer bereits sterilisiert wurde oder nicht. Die Sterilindikatoren reagieren dabei auf Wärme und/oder Dampf und ändern ihre Farbe während des Sterilisationsvorgangs. Nach dem Sterilisationsvorgang kann das Personal anhand der Farbe des Indikatorpunktes feststellen, dass der Inhalt des Containers steril ist. Die Indikatorpunkte sind entweder auf Beschriftungskartons oder auf einer Containerplombe angebracht. Befindet sich der Indikatorpunkt auf einem Beschriftungskarton, besteht aber die Gefahr, dass ein solcher Beschriftungskarton nach der Verwendung eines Containers und dessen Inhalts nicht entfernt und durch einen neuen Beschriftungskarton ersetzt wurde, sondern der Beschriftungskarton an dem Container verbleibt und somit fälschlicher Weise anzeigt, dass der Container mitsamt seinem Inhalt frisch sterilisiert wurde.

Befindet sich der Indikatorpunkt an einer Containerplombe, die zum Öffnen des Containers zerstört werden muss, kann es nicht versehentlich passieren, dass der alte Indikatorpunkt an dem Container verbleibt. Bei einigen Containerplomben kann man aber nicht gut erkennen, ob die Plombe bereit zerstört wurde. In diesem Fall kann eine alte Plombe an einem Container angebracht werden und somit ebenfalls fälschlicher Weise anzeigen, dass der Inhalt des Containers steril sei. In beiden Fällen aber muss der Indikator nach jedem Einsatz erneuert werden, was einen zusätzlichen Arbeitsschritt und die Bevorratung der Indikatoren oder der Plomben mit Indikator erfordert.

Andere Sterilanzeigesysteme arbeiten mit Bimetallfedern oder Formgedächtnismetallen, welche sich während des Sterilisationsvorgangs zumeist gegen eine reguläre Spiralfeder verformen und ein Anzeigeelement verschieben, um es dann unter Vorspannung der regulären Spiralfeder zu verrasten. Wird der Container geöffnet, löst sich die Verrastung des Anzeigeelements und es kehrt in eine Stellung zurück, die nicht mehr den sterilen Zustand anzeigt. Diese Systeme haben aber den Nachteil, dass sie einen sehr komplexen Aufbau benötigen, der viele einzelne bewegliche Teile erfordert. Außerdem erfordern diese Systeme ein beträchtliches Bauvolumen, welches das nutzbare Volumen des Containers verringert. Dies führt zu erheblichen Herstellkosten. Bauteile aus Formgedächtnismetallen sind zudem sehr teuer. Dafür sind diese Systeme reversibel einsetzbar und müssen nicht vor jedem Sterilisationsvorgang ersetzt werden wie die zuvor beschriebenen Indikatorpunkte.

Ebenso sind im Stand der Technik sogenannte Phasenwechselmaterialien (PCM - phase change materials) bekannt. Phasenwechselmaterialien werden üblicherweise für Latentwärmespeicher verwendet. Einen Einblick in die Grundlagen zu Latentwärmespeicher und Phasenwechselmaterialien gibt der Aufsatz "Latentwärmespeicher - Funktionsprinzip und Anwendungsbereiche" von Petra Oberpaul, 2002. Dort sind als Anwendungsgebiete beispielsweise die Solarenergieerzeugung, der Wohnungsbau oder die Kraftfahrzeugindustrie genannt. Darüber hinaus werden drei Klassen von Phasenwechselmaterialien unterschieden, eutektische Wasser-Salz-Lösungen, organische Phasenwechselmaterialien und Salzhydrate. Eutektische Wasser-Salz-Lösungen werden überwiegend zur Kältespeicherung verwendet. Paraffine (langkettige Kohlenwasserstoffe) und Zuckeralkohole, beispielsweise Erythritol, Mannitol und Sorbitol, gehören zu den organischen Phasenwechselmaterialien. In dem angestrebten Temperaturbereich zwischen der Raumtemperatur und der Sterilisationstemperatur sind Salzhydrate die bevorzugte Wahl. Als Beispiele für Salzhydrate seien hier Kalziumchlorid-Hexahydrat, Natriumsulfat-Dekahydrat, Dinatriumhydrogenphosphat-Dodekahydrat, Dinatriumthiosulfat-Pentahydrat, Natriumacetat-Trihydrat, Bariumhydroxid-Oktahydrat und ein Gemisch aus Magnesiumhydrat-Hexahydrat und Litiumnitrat genannt.

Das Dokument WO 01/86289 A1 beschreibt Hitze-Sterilanzeigen, welche auf Farbwechsel durch Isomerisation beruhen.

Die Aufgabe der vorliegenden Erfindung ist es daher, eine reversible Sterilanzeige für einen Sterilisationscontainer bereit zu stellen, die einfach aufgebaut, günstig, platzsparend und reversibel ist. Die Aufgabe der vorliegenden Erfindung wird gelöst durch die Verwendung eines Phasenwechselmaterials zur Herstellung einer Sterilanzeige nach Anspruch 1, eine Sterilanzeige nach Anspruch 3 und einen Sterilisationscontainer nach Anspruch 8. Vorteilhafte Ausgestaltungen der Verwendung und der Vorrichtungen sind Gegenstand der abhängigen Ansprüche.

Gemäß einem ersten Aspekt der vorliegenden Erfindung wird ein Phasenwechselmaterial zur Herstellung einer Sterilanzeige für einen Sterilisationscontainer verwendet. Ein Phasenwechselmaterial ist ein Material, welches sich bei einer bestimmten Temperatur von der festen Phase in die flüssige Phase begibt, d.h. schmilzt und/oder sich löst, und dabei Energie aufnimmt. In der festen Phase weisen Phasenwechselmaterialien eine kristalline Struktur auf und sind daher im Wesentlichen undurchsichtig. In der flüssigen Phase hingegen sind die Phasenwechselmaterialien klar und gut durchsichtig. In der festen Phase kann ein Phasenwechselmaterial demnach den Blick auf ein Anzeigeelement versperren, welches anzeigt, dass der Gegenstand, an dem das Phasenwechselmaterial und die Anzeige vorgesehen sind, steril ist. In der flüssigen Phase hingegen gibt das Phasenwechselmaterial den Blick auf die Anzeige frei und zeigt dem Betrachter damit an, dass der Gegenstand und ggf. dessen Inhalt steril ist. Der Benutzer kann aber auch den sterilen bzw. unsterilen Zustand des Gegenstands direkt daran erkennen, ob das Phasenwechselmaterial flüssig oder fest ist, entweder optisch oder durch ertasten, wenn das Material in einem weichen Behälter aufgenommen ist. Eine Anzeige ist also nicht unbedingt erforderlich. Ebenso wenig ist es unbedingt erforderlich, dass der Behälter einen durchsichtigen Bereich aufweist.

Darüber hinaus weisen die Phasenwechselmaterialien noch eine weitere entscheidende Eigenschaft auf. Phasenwechselmaterialien können in der flüssigen Phase stark unterkühlt werden, also unter den Gefrierpunkt bzw. Erstarrungspunkt abgekühlt werden, ohne dass sie die flüssige Phase selbständig verlassen und erstarren bzw. frieren. Um aus dem unterkühlten Zustand zu kristallisieren, d.h. zu frieren und/oder zu erstarren, muss das Phasenmaterial unter eine Kristallisationstemperatur abgekühlt werden, die weit unter der Gefriertemperatur liegt, es müssen Kristallisationskeime zugegeben werden und/oder es muss eine Aktivierung des Kristallisationsvorganges mittels Druckstoß bzw. Druckwelle oder einem anderen Energieeintrag wie beispielsweise eines elektrischen Funken erfolgen. Dies bedeutet, dass das Phasenwechselmaterial nach einer Verflüssigung lange Zeit bei einer Temperatur, die deutlich unterhalb der Schmelztemperatur des Materials liegt, im flüssigen Zustand verbleiben kann und dann durch eine Aktivierung der Kristallisation gefriert, wobei das Material Wärme abgibt und sich gleichzeitig eintrübt. Ist die Kristallisation einmal angestoßen, friert das komplette Phasenwechselmaterial wie in einer Kettenreaktion durch. Andererseits benötigt das Phasenwechselmaterial eine Wärmezufuhr oberhalb der Schmelztemperatur (identisch mit der Gefriertemperatur) über eine gewisse Zeit, bis es sich vollständig verflüssigt. Die benötigte Zeit hängt von der Menge des Phasenwechselmaterials und der verwendeten Temperatur ab.

Ein Sterilisationsvorgang im medizinischen Bereich, beispielsweise eines mit Instrumenten gefüllten medizinischen Sterilisationscontainers in einem Krankenhaus, spielt sich in einem Temperaturbereich von der Zimmertemperatur bis mindestens 100°C ab. Die Zimmertemperatur kann in verschiedenen Regionen der Welt unterschiedlich sein. Es ist auch möglich, dass die Sterilisation der Geräte nicht an dem Ort erfolgt, an dem diese eingesetzt werden. In diesem Fall kann das Phasenwechselmaterial während des Transports auch Temperaturen ausgesetzt sein, die deutlich unterhalb der Zimmertemperatur liegen, gegebenenfalls auch unter 0°C. Es gibt Phasenwechselmaterialien, die eine Kristallisationstemperatur aufweisen, die bei etwa -20°C liegt. Andererseits gibt es Phasenwechselmaterialien wie z.B. D-Mannitol, die einen Schmelzpunkt (liegt bei ca. 160°C) aufweisen, der deutlich über der Temperatur liegt, die bei einem herkömmlichen Sterilisationsvorgang mittels Dampfsterilisation in einem Krankenhaus erreicht werden.

Dies bedeutet, dass der Schmelzpunkt bzw. die Schmelztemperatur des Phasenwechselmaterials oberhalb der Zimmertemperatur liegen muss. Idealerweise liegt er noch etwas darüber, damit kein unbeabsichtigtes Verflüssigen des Phasenwechselmaterials durch beispielsweise, Sonneneinstrahlung oder die Wärme einer Beleuchtungseinrichtung, durch Anfassen (Körpertemperatur ca. 37°C), durch versehentliche offene Dampfeinwirkung (z.B. aus einer Spülmaschine entweichender Dampf), etc. erfolgt. Dies könnte dazu führen, dass ein unsteriler Container mitsamt Inhalt ungewollt für steril gehalten wird, da sich das Phasenwechselmaterial verflüssigt, ohne dass ein korrekter Sterilisationsvorgang erfolgt ist. Die Kristallisationstemperatur muss aber unterhalb der minimalen Raumtemperatur liegen, damit nicht versehentlich ein steriler Container für unsteril gehalten wird, da das Phasenwechselmaterial die Anzeige für den sterilen Zustand nach einem ordentlichen Sterilisationsvorgang verbirgt, ohne dass der Inhalt des Containers tatsächlich unsteril geworden ist (beispielsweise durch Öffnen des Containers). Zuletzt muss die Schmelztemperatur unter der maximalen Temperatur liegen, die bei einem herkömmlichen Sterilisationsvorgang erreicht wird, da ansonsten keine Verflüssigung des Phasenwechselmaterials während des Sterilisationsvorgangs erfolgt und der Container immer als unsteril gekennzeichnet bleibt. Es muss auch darauf geachtet werden, dass der Wärmeeintrag in das Phasenwechselmaterial während des Sterilisationsvorgangs ausreichend ist, um das gesamte Phasenwechselmaterial zu verflüssigen, da das nicht verflüssigte Phasenwechselmaterial bei einer Abkühlung sonst als Kristallisationskeim für das bereits verflüssigte Phasenwechselmaterial dient und dieses somit sofort beim Abkühlen gefriert und nicht den unterkühlten Zustand erreicht.

**Tabelle 1: Salzhydrate und deren Schmelztemperatur**

| ***Salzhydrat (chemische Formel)*** | ***Schmelztemperatur*** |
|---|---|
| Kalziumchlorid-Hexahydrat (CaCl₂•6H₂O) | 27°C |
| Natriumsulfat-Dekahydrat (Na₂SO₄•10H₂O) | 32°C |
| Dinatriumhydrogenphosphat-Dodekahydrat (Na₂HPO₄•12H₂O) | 35°C |
| Dinatriumthiosulfat-Pentahydrat (Na₂S₂O₃•5H₂O) | 48°C |
| Natriumacetat-Trihydrat (NaCH₃COO•3H₂O) | 58°C |
| Magnesiumhydrat-Hexahydrat/Litiumnitrat (Mg(NO₃)₂•6H₂O/Li(NO₃)) | 72°C |
| Bariumhydroxid-Oktahydrat (Ba(OH)₂•8H₂O) | 78°C |

Gemäß einer vorteilhaften Ausführungsform des ersten Aspekts der vorliegenden Erfindung weist das Phasenwechselmaterial eine Schmelztemperatur in einem Temperaturbereich von 25°C bis 100°C, bevorzugt von 30°C bis 80°C, weiter bevorzugt von 35°C bis 75°C und meist bevorzugt von 40°C bis 60°C auf. In dem erstgenannten Temperaturbereich ist für die untere Grenze eine etwas erhöhte Raumtemperatur berücksichtigt, die beispielsweise an warmen Sommertagen erreicht werden kann, sodass kein unbeabsichtigtes Verflüssigen des verwendeten Phasenwechselmaterials erfolgen kann. Die obere Grenze ist so gewählt, dass eine vollständige Verflüssigung des Phasenwechselmaterials bei jedem derzeit in einem Krankenhaus eingesetzten Sterilisationsvorgang sicher gestellt ist. Die zweit- und drittgenannten Temperaturbereiche erhöhen schrittweise die Sicherheit gegenüber einer unerwünschten Verflüssigung des Phasenwechselmaterials einerseits und verhindern andererseits, dass sich das Personal an dem frisch sterilisierten Container verletzt (verbrennt), wenn dieser nach einem Sterilisationsvorgang aus dem Sterilisator entnommen wird. Der letztgenannte Bereich ermöglicht es dem Personal zudem, dass es den Container ungeschützt aus dem Sterilisator entnehmen kann. Es muss hier auch berücksichtigt werden, dass ein Öffnen des Containers oder seines Verschlusses zu einer Kristallisation des Phasenwechselmaterials führt. Dabei erreicht das Material seine Schmelztemperatur und behält die aufgrund der relativ großen Energiedichte der Phasenwechselmaterialien relativ lange bei, bevor es auf die Raumtemperatur abkühlt. Bei hohen Schmelztemperaturen besteht also die Gefahr einer Verletzung (z.B. Verbrennung) des Personals auch dann, wenn der Container bereits abgekühlt ist. Durch die Kristallisation erwärmt bzw. erhitzt sich der Container lokal auf die Schmelztemperatur. Eine niedrigere Schmelztemperatur erhöht also die Sicherheit des Personals bei der Verwendung des Containers. Zudem verkürzt eine niedrige Schmelztemperatur die Zeit erheblich, bis sich das Phasenwechselmaterial nach der Kristallisation wieder auf die Raumtemperatur abkühlt. Das Phasenwechselmaterial kann insbesondere Natriumacetat-Trihydrat und/oder Dinatriumthiosulfat-Pentahydrat enthalten. Es gibt aber auch organische Phasenwechselmaterialien wie D-Sorbitol, deren Schmelztemperatur sich in einem komfortablen und sicheren Bereich befindet (ca. 70°C). Eine Verletzung des Personals bei oder kurz nach der Kristallisation des Phasenwechselmaterials kann aber auch konstruktiv verhindert werden, indem die Wärme von möglichen Kontaktbereichen mit dem Personal weg geleitet wird, indem die Menge des Phasenwechselmaterials möglichst gering gehalten wird und/oder indem das Phasenwechselmaterial vor einem direkten Kontakt abgeschirmt und/oder isoliert wird.

Gemäß einem zweiten Aspekt der vorliegenden Erfindung ist eine Sterilanzeige für einen Sterilisationscontainer offenbart mit einem fluiddichten Behälter, der ein Phasenwechselmaterial enthält. Die Behälterwand des Behälters weist zumindest einen transparenten Bereich auf, sodass man von außen in den Behälter sehen kann. Die Sterilanzeige weist zudem einen Aktivator auf, welcher betätigbar ist, um eine Aktivierungsenergie und/oder Kristallisationskeime frei zu setzen. Der Aktivator befindet sich in Kontakt mit dem Phasenwechselmaterial und eine Betätigung des Aktivators gibt eine Aktivierungsenergie und/oder Kristallisationskeime an das unterkühlte und flüssige Phasenwechselmaterial frei, woraufhin dieses friert bzw. sich verfestigt. Beim Frieren bzw. Verfestigen des Phasenwechselmaterials wird Wärme freigesetzt und vor allem verändert sich das Phasenwechselmaterial von einer klaren (transparenten) Flüssigkeit in einen opaken (intransparenten) Festkörper. Teilweise kann dieser Übergang über einen Mischzustand erfolgen, in dem ein Teil des Phasenwechselmaterials noch in der flüssigen Phase vorliegt, welche sich in einem bereits erstarrten Kristallgitter befindet. In diesem Zwischenzustand ist das Phasenwechselmaterial aber bereits opak. In dieser grundlegenden Ausführungsform kann ein Nutzer der Sterilanzeige durch den transparenten Bereich der Behälterwand erkennen, ob sich eine klare (transparente) Flüssigkeit in dem Behälter befindet oder ob sich ein opaker Festkörper darin befindet. Der Zwischenzustand ist ebenfalls erkennbar. Nähere Details zu dem Zwischenzustand können dem Stand der Technik entnommen werden.

Gemäß einer vorteilhaften Ausführungsform des zweiten Aspekts der vorliegenden Erfindung ist eine Beschriftung und/oder farbliche Kennzeichnung an und/oder in dem fluiddichten Behälter so vorgesehen, dass sie dem transparenten Bereich der Behälterwand zumindest teilweise zugewandt ist. Diese Ausführungsform hat den Vorteil, dass der Nutzer nicht die Transparenz oder Intransparenz des Phasenwechselmaterials selbst erkennen muss, sondern dass es sich die Transparenz des flüssigen Materials zu Nutze machen kann und eine Anzeige erkennen kann, die in dem Behälter oder an dessen Innenwand vorgesehen ist. Es ist einfacher für einen Nutzer zu erfassen, ob eine farbliche Kennzeichnung oder eine Beschriftung erkennbar ist oder nicht, als zu erfassen, ob ein Medium in einem Behälter transparent oder opak ist. Insbesondere eine Kombination von farblicher Kennzeichnung und Beschriftung wie beispielsweise der Schriftzug "steril" oder "sterile" in Schwarzen Buchstaben auf einer grünen Fläche sollte für einen Nutzer durch das flüssige und somit transparente bzw. klare Phasenwechselmaterial leicht zu erkennen sein. Ist eine solche Kennzeichnung nicht erkennbar, befindet sich das Phasenwechselmaterial nicht in der flüssigen Phase sondern in der festen Phase oder einem Übergangszustand.

Gemäß einer anderen vorteilhaften Ausführungsform des zweiten Aspekts der vorliegenden Erfindung ist die Beschriftung und/oder farbliche Kennzeichnung an einer Innenseite der Behälterwand vorgesehen und bevorzugt dem transparenten Bereich der Behälterwand gegenüberliegend angeordnet. In diesem Fall kann die Anzahl der Bauteile gering gehalten werden. Idealerweise besteht der Behälter aus einer transparenten vorderen Halbschale, einer hinteren Halbschale mit im Wesentlichen spielgelsymmetrischer Form und einer Kennzeichnung und/oder Beschriftung an deren Innenwand. Der Aktivator ist in einer Aussparung vorgesehen, die an einer Stelle in einem Kontaktbereich der beiden Halbschalen des Behälters vorgesehen ist. Ein solcher Behälter ist leicht, schnell, günstig und in geringer Größe herstellbar und verarbeitbar. Weiter bevorzugt ist der Bereich der Behälterwand, der die Beschriftung und/oder farbliche Kennzeichnung aufweist, und/oder der transparente Bereich der Behälterwand im Wesentlichen eben und/oder gewölbt ausgebildet. Mit einer solchen Ausbildung macht man sich einen Lupeneffekt zu Nutze, der entsteht, wenn sich das Phasenwechselmaterial in der flüssigen Phase befindet. Dies macht es für den Nutzer noch einfacher, die Kennzeichnung und/oder die Beschriftung zu erkennen, wenn das Phasenwechselmaterial flüssig ist.

Gemäß noch einer vorteilhaften Ausführungsform des zweiten Aspekts der vorliegenden Erfindung weist der Aktivator ein Federelement nach dem Knackfroschprinzip auf, welcher bevorzugt in der Behälterwand des fluiddichten Behälters angeordnet ist. Idealerweise weist das Federelement eine rotationssymmetrische Form mit einer zentralen Wölbung auf. Auf diese Weise kann das Federelement als ein Teil der Behälterwand ausgebildet sein und zwischen zwei im Wesentlichen Halbschalen des Behälters angeordnet sein. Der Rand des Federelements kann dabei fest durch die Behälterwand gehalten sein, da sich der Rand des Federelements bei einer Aktivierung nicht oder nur unwesentlich verformt. Die zentrale Wölbung hingegen kann sich in den Container hinein oder aus diesem heraus bewegen und dabei den Phasenwechsel des Phasenwechselmaterials von flüssig nach fest auslösen.

Wenn das Federelement die Kristallisation des Phasenwechselmaterials durch ein Bereitstellen von Kristallisationskeimen auslöst, so sind diese Kristallisationskeime nach aktuellem Stand der Wissenschaft keine Bruchstücke oder dergleichen des Federelements. Vielmehr ist davon auszugehen, dass die Oberfläche des Federelements winzige Furchen und/oder Spalten aufweist, in denen bei einer Biegung des Materials winzige Mengen von kristallisiertem Material eingeschlossen werden und auf diese Weise, wenn das Federelement in seine Ausgangsposition zurück kehrt, vom Rest des Phasenwechselmaterials getrennt werden. Diese Kristallisationskeime, die in dem Federelement gefangen sind, verflüssigen sich auch dann nicht, wenn sich der Rest des Phasenwechselmaterials durch entsprechende Wärmezufuhr verflüssigt. Dies kann beispielsweise daran liegen, dass das Volumen bei der Verflüssigung des Phasenwechselmaterials zunimmt. In dem Federelement kann dies beispielsweise durch die vorherrschenden Druckverhältnisse verhindert sein. Wird das Federelement nun erneut gebeugt bzw. gebogen, kommen die bislang abgetrennten Bereiche mit verfestigtem Phasenwechselmaterial mit dem übrigen, flüssigen Phasenwechselmaterial in Kontakt und das flüssige Phasenwechselmaterial kristallisiert durch. Für die Funktionsweise der Sterilanzeige spielt der Auslösemechanismus im Detail keine Rolle. Auch Bruchstücke des Federelements sind in der Lage, die Kristallisation des flüssigen Phasenwechselmaterials auszulösen. Abgesehen davon gibt es auch Hinweise darauf, dass die durch den Knackfroscheffekt bewirkte Druckwelle (Schallwelle des Knackens) für einen Start des Kristallisationsvorgangs ausreichend ist. Die Kristallisation kann mit einem Federelement, welches den Knackfroscheffekt nutzt, sicher und reproduzierbar ausgelöst werden. Die weit verbreiteten Handwärmer mit/aus Phasenwechselmaterial verwenden allesamt einen Aktivator nach diesem Prinzip.

Gemäß einer weiteren vorteilhaften Ausführungsform des zweiten Aspekts der vorliegenden Erfindung weist der Aktivator ein Piezoelement auf, welches daran angepasst ist, die erzeugte elektrische Energie an das Phasenwechselmaterial abzugeben. Neben der Bereitstellung von Kristallisationskeimen und dem Einbringen von Druckwellen und/oder Schallwellen in das Phasenwechselmaterial kann die Kristallisation auch durch einen elektrischen Impuls ausgelöst werden. Besonders einfach kann ein solcher elektrischer Impuls mittels eines Piezoelements bereit gestellt werden, wie es in ähnlicher Weise auch bei Gasfeuerzeugen zum Einsatz kommt. Ein solcher Aktivator ist günstig und von geringer Größe und kann leicht verarbeitet werden.

Gemäß einer anderen vorteilhaften Ausführungsform des zweiten Aspekts der vorliegenden Erfindung ist die Sterilanzeige an entweder der Containerwanne oder dem Containerdeckel vorgesehen und der Aktivator steht im verschlossenen Zustand des Sterilisationscontainers mittelbar oder unmittelbar mit dem anderen von Containerwanne und Containerdeckel in Verbindung. Mit einer solchen Ausgestaltung kann die Anzahl an Bauteilen minimiert werden und sicher gestellt werden, dass beim Öffnen des Sterilisationscontainers der Aktivator ausgelöst bzw. betätigt wird und somit eine Kristallisation des flüssigen Phasenwechselmaterials ausgelöst wird. Ein Öffnen des Sterilisationscontainers bedeutet hierbei entweder, dass der Deckel von der Wanne des Sterilisationscontainers abgehoben bzw. entfernt wird.

Gemäß noch einer weiteren vorteilhaften Ausführungsform des zweiten Aspekts der vorliegenden Erfindung weist der Containerdeckel oder die Containerwanne eine schwenkbare Verschlusslasche auf und der Aktivator steht im verschlossenen Zustand der Verschlusslasche mittelbar oder unmittelbar mit der Verschlusslasche in Verbindung. Dabei kommt es nicht darauf an, ob die Verschlusslasche schwenkbar an dem Containerdeckel oder der Containerwanne vorgesehen ist. Bei einer solchen Ausführungsform kann ein Öffnen des Sterilisationscontainers und somit ein Auslösen der Kristallisation des flüssigen Phasenwechselmaterials bereits bewirkt werden, wenn die Verschlusslasche aus ihrer Verschlussposition heraus bewegt wird. Dazu steht der Aktivator mit der Verschlusslasche in Verbindung und wird bei deren Betätigung bzw. Bewegung ausgelöst.

Gemäß einer weiteren vorteilhaften Ausführungsform des zweiten Aspekts der vorliegenden Erfindung ist der Aktivator ein Federelement nach dem Knackfroschprinzip, welcher bevorzugt in der Behälterwand des fluiddichten Behälters angeordnet ist, wobei sich das Federelement im verschlossenen Zustand der Verschlusslasche und/oder des Sterilisationscontainers bevorzugt in einem vorgespannten Zustand befindet. Auf diese Weise löst auch ein sehr langsames und behutsames Öffnen des Sterilisationscontainers die Kristallisation des flüssigen Phasenwechselmaterials aus, indem das Federelement sich schlagartig entspannt, wenn der dafür benötigte Raum freigegeben wird. Je nachdem an welchem Bauteil die Sterilanzeige vorgesehen ist, wird der Containerdeckel, die Containerwanne oder die Verschlusslasche an einem der beiden vorgenannten Bauteile von dem Federelement weg bewegt und gibt den zu Entspannung des Federelements erforderlichen Raum frei. Beim Entspannen des Federelements wird einerseits eine Druckwelle verursacht, welche eine Kristallisation des flüssigen Phasenwechselmaterials auslöst, andererseits werden winzige, in dem Federelement eingeschlossene Kristallisationskeime freigesetzt, welche ebenfalls eine Kristallisation des flüssigen Phasenwechselmaterials bewirken. Die genauen Vorgänge zur Auslösung der Kristallisation des Phasenwechselmaterials sind im vorstehend aufgeführten Stand der Technik beschrieben.

Gemäß noch einer weiteren vorteilhaften Ausführungsform des zweiten Aspekts der vorliegenden Erfindung steht das Federelement im verschlossenen Zustand der Verschlusslasche mittels eines vorzugsweise elastisch gelagerten Betätigungselements mit der Verschlusslasche und insbesondere einer Seitenfläche der Verschlusslasche in Verbindung, sodass es bei einer Betätigung der Verschlusslasche von dem Behälter weg bewegt wird. Dadurch kann sicher gestellt werden, dass sich das Betätigungselement auch bei längerer Lagerung des Sterilisationscontainers im sterilisierten Zustand nicht festsetzt und somit die Kristallisation des flüssigen Phasenwechselmaterials fehlerhafterweise nicht ausgelöst wird.

Weitere Vorteile und Merkmale der Erfindung sind dem Fachmann aus den beigefügten Figuren und der detaillierten Beschreibung der Ausführungsbeispiele ersichtlich.
- Fig. 1: zeigt eine isometrische Ansicht der Sterilanzeige gemäß dem bevorzugten Ausführungsbeispiel im sterilen Zustand;
- Fig. 2: zeigt eine isometrische Ansicht der Sterilanzeige gemäß dem bevorzugten Ausführungsbeispiel im unsterilen Zustand;
- Fig. 3: zeigt eine Ansicht der Sterilanzeige gemäß dem bevorzugten Ausführungsbeispiel im sterilen Zustand von vorn; und
- Fig. 4: zeigt eine Ansicht der Sterilanzeige gemäß dem bevorzugten Ausführungsbeispiel und einer Verschlusslasche eines Sterilisationscontainers im sterilen Zustand von vorn.

Ein bevorzugtes Ausführungsbeispiel der vorliegenden Erfindung ist unter Bezugnahme auf die Figuren im Folgenden im Detail beschrieben.

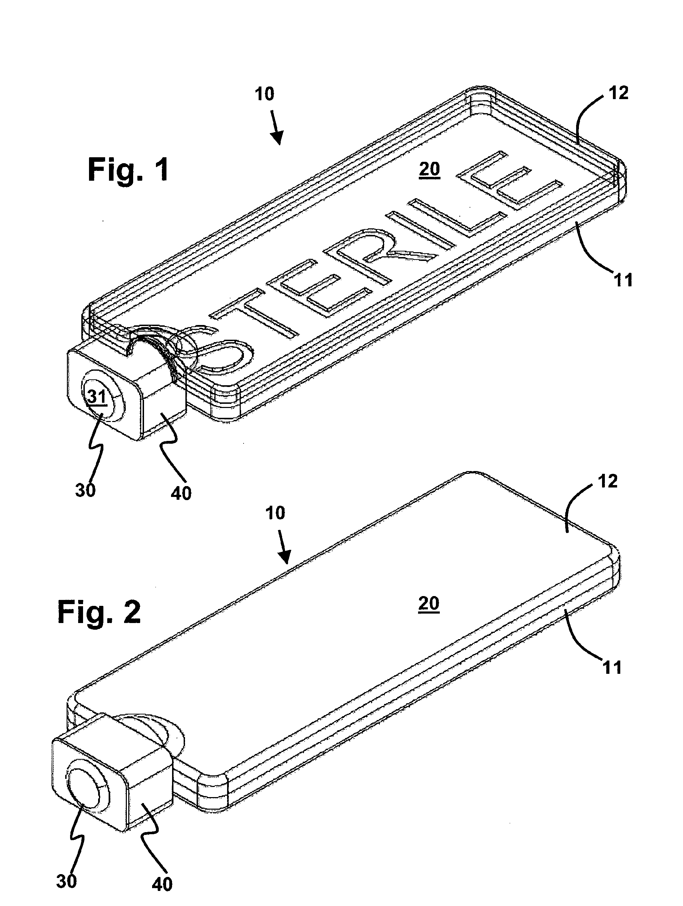

Die Fig. 1 zeigt eine Sterilanzeige für einen Sterilisationscontainer (nicht gezeigt) mit einem fluiddichten Behälter 10, der ein Phasenwechselmaterial 20 enthält. Der Behälter 10 ist aus zwei schalenförmigen Bauteilen 11 und 12 gebildet, wobei das Bauteil 11 die Rückwand 11 des Behälters 10 bildet, an welchem der Behälter an der Wanne des Sterilisationscontainers befestigt ist, und das Bauteil 12 die vordere Wand 12 bildet, die dem Nutzer zugewandt ist. Die vordere Wand 12 ist aus transparentem Kunststoff ausgebildet und die Rückwand 12 ist aus intransparentem Kunststoff ausgebildet. An der Vorderseite der Rückwand 11 ist ein Schriftzug "sterile" ausgebildet, beispielsweise als schwarzer Schriftzug auf hellgrünem Hintergrund. Wenn das Phasenwechselmaterial 20 flüssig ist, ist dieser Schriftzug "sterile" für den Nutzer lesbar. Zwischen der Rückwand 11 und der vorderen Wand 12 ist ein rundes Metallplättchen 15 vorgesehen, welches im entspannten Zustand gewölbt ist und somit einen sogenannten Knackfrosch bildet. Die Rückwand 11, die vordere Wand 12 und das Metallplättchen 15 schließen das Phasenwechselmaterial 20 fluiddicht ein. Zudem füllt das Phasenwechselmaterial den Behälter 10 vollständig aus. Da sich das Phasenwechselmaterial bei der Kristallisation ausdehnt, ist der Behälter so elastisch, dass er durch diese Volumenzunahme nicht beschädigt wird. Alternativ kann an dem Behälter ein Abschnitt vorgesehen sein, der dem Ausgleich der Volumenzunahme dient, beispielsweise ein Kunststoffbeutel. Damit der Behälter 10 im Wesentlichen dünn gestaltet werden kann, ohne dass das Metallplättchen 15 zu klein ausgebildet werden muss, weisen die Rückwand 11 und die vordere Wand 12 Bereiche (für die Rückwand nicht gezeigt) auf, welche sich aufwölben.

Das Metallplättchen 15 ist im vorgespannten Zustand im Wesentlichen eben und befindet sich in Kontakt mit einem Betätigungselement 30. Das Betätigungselement 30 ist in einem Lagerungselement 40 gleitend so gelagert, dass es von dem Metallplättchen 15 weg und zu diesem hin bewegbar ist. Eine Feder 35 stützt sich einerseits an dem Lagerungselement 40 und andererseits an dem Betätigungselement 30 ab und drängt das Betätigungselement 30 von dem Metallplättchen 15 weg.

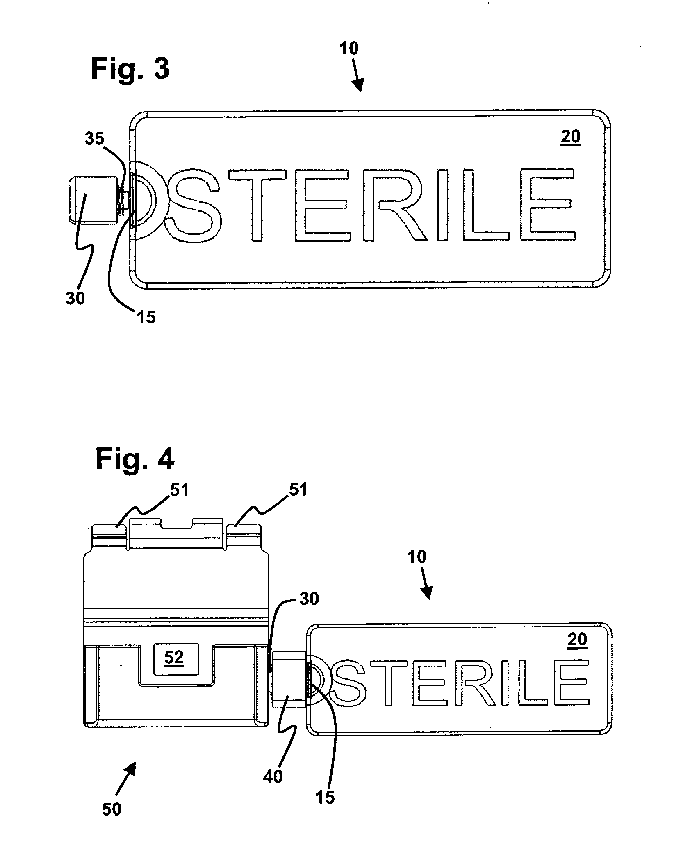

Das Metallplättchen 15 ist durch das Ende des Betätigungselements 30 zum Behälterinneren hin vorgespannt, da das andere End durch eine Verschlusslasche 50 des Sterilisationscontainers, welche an dem Containerdeckel schwenkbar angebracht ist, gegen die Kraft der Feder 35 zu dem Behälter 10 hin gedrängt wird. In der in Fig. 4 gezeigten Ansicht befindet sich die Verschlusslasche 50 in dem geschlossenen Zustand. Die Verschlusslasche 50 ist mittels zwei Ösenelementen 51, 51 an einer nicht gezeigten Lasche schwenkbar an dem Containerdeckel gelagert. In der Öffnung 52 befindet sich ein an der Containerwanne vorgesehener Vorsprung. Die Verschlusslasche 50 entspricht im Prinzip einer Verschlusslasche, wie sie im Stand der Technik gezeigt ist.

In der Fig. 4 befindet sich die Verschlusslasche 50 im geschlossenen Zustand und die Sterilanzeige zeigt an, dass der Container steril ist, da das Phasenwechselmaterial 20 in dem Behälter 10 flüssig ist. Dies bedeutet, dass der Container seit dem letzten Verschließen der Verschlusslasche 50 einen Sterilisationsvorgang durchlaufen hat, während dem der Sterilisationscontainer und somit auch die Sterilanzeige über eine gewisse Zeit einer gewissen Temperatur ausgesetzt waren. Im vorliegenden Ausführungsbeispiel ist Natriumacetat-Trihydrat (NaCH₃COO•3H₂O) als Phasenwechselmaterial 20 in dem Behälter 10 vorgesehen. Der Sterilisationsvorgang erreicht weit über 58°C, was der Schmelztemperatur des Natriumacetat-Trihydrats entspricht, und hält diese Temperatur über eine ausreichend lange Zeit, sodass eine vollständige Verflüssigung des in dem Behälter 10 befindlichen Natriumacetat-Trihydrat 20 erreicht wird. Unberührt hiervon bleiben gegebenenfalls die Kristallisationskeime, die in minimalen Kerben eingeschlossen sind, die in dem Metallplättchen 15 ausgebildet sind. Nach dem Sterilisationsvorgang kühlt der Container durch die geringere Umgebungstemperatur auf eine Temperatur unter 58°C ab und das Natriumacetat-Trihydrat gelangt in einen metastabilen unterkühlten Zustand. In diesem unterkühlten Zustand bleibt das Natriumacetat-Trihydrat zunächst flüssig und transparent.

Wird nun der Inhalt des Sterilisationscontainers benötigt, so wird der Container daraufhin geprüft, ob er noch steril ist. Die Sterilanzeige zeigt eindeutig den Schriftzug "sterile", sodass der Nutzer sicher sein kann, dass der Inhalt des Containers steril ist. Wird nun die Verschlusslasche betätigt, drängt die Feder 35 das Betätigungselement 30 von dem Lagerungselement 50 und dem Behälter 10 weg. Das vorgespannte Metallplättchen 15 unterstützt diese Bewegung und der Knackfroscheffekt des Metallplättchens wird ausgelöst. Dies löst eine Kristallisation des Natriumacetat-Trihydrats aus und es wird binnen wenigen Sekunden undurchsichtig. Ab nun ist der Schriftzug "steril" der Sterilanzeige nicht mehr zu erkennen, wie dies in der Fig. 2 gezeigt ist. Der Deckel des Containers kann nun abgenommen werden und die in der Containerwanne bzw. einem darin vorgesehenen Siebkorb enthaltenen Instrumente und/oder Implantate können verwendet werden. Nach der Verwendung werden die Instrumente und ggf. auch die Implantate wieder in die Containerwanne bzw. einen in der Containerwanne vorgesehenen Siebkorb zurück gelegt und der Deckel des Containers wird wieder auf die Containerwanne aufgesetzt. Anschließend wird die Verschlusslasche in den verschlossenen Zustand gebracht, sodass der Sterilisationscontainer korrekt verschlossen ist.

Hierdurch wird aber dem Natriumacetat-Trihydrat nicht genug Energie zugeführt, als dass sich dieses verflüssigen würde und somit den Schriftzug "sterile" wieder sichtbar machen würde. Da die Sterilanzeige zu diesem Zeitpunkt also nicht den Schriftzug "sterile" erkennen lässt, weiß nun jeder weitere potentielle Nutzer, dass der Inhalt des Sterilisationscontainers nicht steril ist und nicht verwendet werden darf, bis eine Reinigung und vor allem eine Sterilisation des Containers und dessen Inhalt erfolgt ist.

Auch bei einer Bestrahlung durch starke Lampen und eine erhöhte Raumtemperatur tritt keine vollständige Verflüssigung des Natriumacetat-Trihydrats ein. Bei einer nur teilweisen Verflüssigung des Natriumacetat-Trihydrats, wie es theoretisch geschehen könnte, wenn eine noch heiße Elektrode eines TFT-Geräts (TFT: Tissue-Fusion-Techology) mit dem Behälter in Berührung käme, kristallisiert das Natriumacetat-Trihydrat anschließend selbständig wieder aus, wenn die lokale Temperatur unter die Schmelztemperatur absinkt.

Dem Fachmann ergeben sich zahlreiche Abwandlungen und Varianten zu dem vorstehend beschriebenen Ausführungsbeispiel. So kann unter Umständen das Phasenwechselmaterial eingefärbt sein oder die vordere Wand 12 des Behälters 10 kann so geformt sein, dass das Phasenwechselmaterial und der transparente Bereich des Behälters 10 eine Art Lupe bilden, die den Schriftzug "sterile" vergrößert darstellen und somit besser lesbar machen.

## Patentansprüche

1. Verwendung eines Phasenwechselmaterials (20) zur Herstellung einer Sterilanzeige für einen Sterilisationscontainer.

2. Verwendung nach Anspruch 1, wobei das Phasenwechselmaterial (20) eine Schmelztemperatur in einem Temperaturbereich von 25°C bis 100°C, bevorzugt von 30°C bis 80°C, weiter bevorzugt von 35°C bis 75°C und meist bevorzugt von 40°C bis 60°C aufweist, wobei das Phasenwechselmaterial (20) insbesondere Natriumacetat-Trihydrat und/oder Dinatriumthiosulfat-Pentahydrat enthält.

3. Sterilanzeige für einen Sterilisationscontainer,
**gekennzeichnet durch**
einen fluiddichten Behälter (10), der ein Phasenwechselmaterial (20) enthält, wobei die Behälterwand des Behälters (10) zumindest einen transparenten Bereich (12) aufweist, und
einen Aktivator (15), welcher betätigbar ist, um eine Aktivierungsenergie und/oder Kristallisationskeime frei zu setzen, wobei sich der Aktivator (15) mit dem Phasenwechselmaterial (20) in Kontakt befindet.

4. Sterilanzeige gemäß Anspruch 3, wobei
eine Beschriftung und/oder farbliche Kennzeichnung an und/oder in dem fluiddichten Behälter (10) so vorgesehen ist, dass sie dem transparenten Bereich (12) der Behälterwand (11, 12) zumindest teilweise zugewandt ist.

5. Sterilanzeige gemäß Anspruch 4, wobei
die Beschriftung und/oder farbliche Kennzeichnung an einer Innenseite der Behälterwand (11) vorgesehen ist und bevorzugt dem transparenten Bereich (12) der Behälterwand gegenüberliegend angeordnet ist, wobei weiter bevorzugt der Bereich der Behälterwand, der die Beschriftung und/oder farbliche Kennzeichnung aufweist, und/oder der transparente Bereich der Behälterwand im Wesentlichen eben und/oder gewölbt ausgebildet ist.

6. Sterilanzeige gemäß einem der Ansprüche 3 bis 5, wobei
der Aktivator (15) ein Federelement nach dem Knackfroschprinzip aufweist, welches bevorzugt in der Behälterwand (11, 12) des fluiddichten Behälters (10) angeordnet ist.

7. Sterilanzeige gemäß einem der Ansprüche 3 bis 6, wobei
der Aktivator (15) ein Piezoelement aufweist, welches daran angepasst ist, die erzeugte elektrische Energie an das Phasenwechselmaterial (20) abzugeben.

8. Sterilisationscontainer mit einer Containerwanne und einem Containerdeckel,
**gekennzeichnet durch**
eine Sterilanzeige nach einem der Ansprüche 3 bis 7, wobei die Sterilanzeige an entweder der Containerwanne oder dem Containerdeckel vorgesehen ist und der Aktivator (15) im verschlossenen Zustand des Sterilisationscontainers mittelbar oder unmittelbar mit dem anderen von Containerwanne und Containerdeckel in Verbindung steht.

9. Sterilisationscontainer nach Anspruch 8, wobei
der Containerdeckel oder die Containerwanne eine schwenkbare Verschlusslasche (50) aufweist und der Aktivator (15) im verschlossenen Zustand der Verschlusslasche (50) mittelbar oder unmittelbar mit der Verschlusslasche (50) in Verbindung steht.

10. Sterilisationscontainer nach Anspruch 8 oder 9, wobei
der Aktivator (15) ein Federelement nach dem Knackfroschprinzip ist, welcher bevorzugt in der Behälterwand (11, 12) des fluiddichten Behälters (10) angeordnet ist, wobei sich das Federelement (15) im verschlossenen Zustand der Verschlusslasche (50) und/oder des Sterilisationscontainers bevorzugt in einem vorgespannten Zustand befindet.

11. Sterilisationscontainer nach Anspruch 10, wobei
das Federelement (15) im verschlossenen Zustand der Verschlusslasche (50) mittels eines vorzugsweise elastisch gelagerten Betätigungselements (30) mit der Verschlusslasche (50) und insbesondere einer Seitenfläche der Verschlusslasche (50) in Verbindung steht.

## Claims

1. Use of a phase change material (20) for the production of a sterile state indication means for a sterilization container.

2. The use according to claim 1, wherein the phase change material (20) has a melting temperature in a temperature range from 25°C to 100°C, preferably from 30°C to 80°C, more preferably from 35°C to 75°C, and most preferably from 40°C to 60°C, the phase change material (20) containing in particular sodium acetate trihydrate and/or disodium thiosulfate pentahydrate.

3. A sterile state indication means for a sterilization container,
**characterized by**
a fluid-tight case (10) containing a phase change material (20), wherein the case wall of the case (10) comprises at least one transparent zone (12), and
an activator (15) which can be actuated in order to release an activation energy and/or crystallization seeds, the activator (15) being in contact with the phase change material (20).

4. The sterile state indication means according to claim 3, wherein
a labeling and/or color marking is provided on and/or in the fluid-tight case (10) in such a manner that it faces the transparent zone (12) of the case wall (11, 12) at least in parts.

5. The sterile state indication means according to claim 4, wherein
the labeling and/or color marking is provided on an inner side of the case wall (11) and is preferably arranged so as to be opposite the transparent zone (12) of the case wall, and more preferably the zone of the case wall comprising the labeling and/or color marking, and/or the transparent zone of the case wall is formed to be substantially planar and/or curved.

6. The sterile state indication means according to one of the preceding claims 3 to 5, wherein
the activator (15) comprises a spring element, which is realized according to the clicker principle and is preferably arranged in the case wall (11, 12) of the fluid-tight case (10).

7. The sterile state indication means according to one of the preceding claims 3 to 6, wherein
the activator (15) comprises a piezo element which is adapted to deliver the produced electric energy to the phase change material (20).

8. A sterilization container comprising a container trough and a container lid,
**characterized by**
a sterile state indication means according to one of claims 3 to 7, wherein the sterile state indication means is provided either on the container trough or on the container lid, and the activator (15), in the closed state of the sterilization container, is in direct or indirect connection with the respectively other element among container trough and container lid.

9. The sterilization container according to claim 8, wherein
the container lid or the container trough comprises a pivotable closure lug (50) and the activator (15), in the closed state of the closure lug (50), is in direct or indirect connection with the closure lug (50).

10. The sterilization container according to claim 8 or 9, wherein
the activator (15) is a spring element which is realized according to the clicker principle and preferably arranged in the case wall (11, 12) of the fluid-tight case (10), the spring element (15) preferably being in a pretensioned state in the closed state of the closure lug (50) and/or of the sterilization container.

11. The sterilization container according to claim 10, wherein
the spring element (15), in the closed state of the closure lug (50), is in connection with the closure lug (50) and in particular with a lateral surface of the closure lug (50) by means of a preferably elastically supported actuation element (30).

## Revendications

1. Utilisation d'un matériau à changement de phase (20) pour la fabrication d'un indicateur de stérilité destiné à un conteneur de stérilisation.

2. Utilisation selon la revendication 1, où le matériau à changement de phase (20) présente une température de fusion comprise dans une plage de température de 25 °C à 100 °C, avantageusement de 30 °C à 80 °C, de préférence de 35 °C à 75 °C et tout particulièrement de 40 °C à 60 °C, le matériau à changement de phase (20) contenant en particulier de l'acétate de sodium trihydraté et/ou du thiosulfate de sodium pentahydraté.

3. Indicateur de stérilité pour un conteneur de stérilisation,
**caractérisé par**
un récipient étanche aux fluides (10) contenant un matériau à changement de phase (20), la paroi du récipient (10) présentant au moins une zone transparente (12), et
un activateur (15) pouvant être actionné pour libérer une énergie d'activation et/ou des germes de cristallisation, l'activateur (15) étant en contact avec le matériau à changement de phase (20).

4. Indicateur de stérilité selon la revendication 3, où une inscription et/ou un marquage de couleur sont prévus sur et/ou dans le récipient étanche aux fluides (10) de manière à être au moins en partie orientés vers la zone transparente (12) de la paroi du récipient (11, 12).

5. Indicateur de stérilité selon la revendication 4, où
l'inscription et/ou le marquage de couleur sont prévus sur une face intérieure de la paroi du récipient (11), et de préférence disposés en face de la zone transparente (12) de la paroi du récipient, et où en outre la zone de la paroi du récipient présentant l'inscription et/ou le marquage de couleur, et/ou la zone transparente de la paroi du récipient sont de préférence sensiblement planes et/ou incurvées.

6. Indicateur de stérilité selon l'une des revendications 3 à 5, où
l'activateur (15) comporte un élément élastique conçu comme ressort à pression à déclic, lequel est disposé de préférence dans la paroi (11, 12) du récipient étanche aux fluides (10).

7. Indicateur de stérilité selon l'une des revendications 3 à 6, où
l'activateur (15) comporte un élément piézo-électrique adapté pour transmettre l'énergie électrique générée au matériau à changement de phase (20).

8. Conteneur de stérilisation avec un bac de conteneur et un couvercle de conteneur,
**caractérisé par**
un indicateur de stérilité selon l'une des revendications 3 à 7, ledit indicateur de stérilité étant prévu soit sur le bac de conteneur soit sur le couvercle de conteneur et l'activateur (15) étant directement ou indirectement relié à l'autre composant de conteneur - bac ou couvercle - en état de fermeture du conteneur de stérilisation.

9. Conteneur de stérilisation selon la revendication 8, où
le couvercle de conteneur ou le bac de conteneur comporte une languette de fermeture (50) pivotante et où l'activateur (15) est directement ou indirectement relié à languette de fermeture (50) en état de fermeture de la languette de fermeture (50).

10. Conteneur de stérilisation selon la revendication 8 ou 9, où
l'activateur (15) est un élément élastique conçu comme ressort à pression à déclic, lequel est disposé de préférence dans la paroi (11, 12) du récipient étanche aux fluides (10), l'élément élastique (15) se trouvant de préférence dans un état de précontrainte en état de fermeture de la languette de fermeture (50) et/ou du conteneur de stérilisation.

11. Conteneur de stérilisation selon la revendication 10, où
l'élément élastique (15) étant en état de fermeture de la languette de fermeture (50) relié à la languette de fermeture (50) et en particulier à une surface latérale de la languette de fermeture (50) au moyen d'un élément d'actionnement (30) monté de préférence sur palier élastique.
